# EUROPEAN PATENT APPLICATION

(11) **EP 2 597 148 A1**
(43) Date of publication of application: **29.05.2013**
(21) Application number: 11191000.6
(22) Date of filing: 28.11.2011
(51) Int. Cl.: C12M 3/04

(54) **Oxygraph chamber and insertable micro-culture device and uses thereof**

(71) Applicant: Consiglio Nazionale Delle Ricerche, 00185 Roma (IT)
(72) Inventor: Aloisi, Alessandra, 73044 Galatone, (LE) (IT); Tarentini, Elisabetta, 73010 Veglie (LE) (IT); Leopizzi, Giovanni, 73052 Parabita (LE) (IT); Rinaldi, Rosaria, 73100 Lecce (LE) (IT); Ferramosca, Alessandra, 73052 Parabita (LE) (IT); Zara, Vincenzo, 73100 Lecce (LE) (IT)
(74) Representative: Capasso, Olga

(57) **Abstract**

The present invention refers to a micro culture device (5) to be inserted in a chamber (4) of an oxygraph (7). The micro culture device is divided into an upper microchamber (2) and a lower microchamber (3) by a semi-permeable nanoporous membrane (1), suitable for acting as adhering support for cells. The upper microchamber (2) presents at its upper side either an inlet (2.1) coaxially aligned with an oxygraph plunger precision bore (6), or a semi-permeable membrane. The lower microchamber (3) presents at its bottom side either an inlet (3.1) coaxially aligned with an oxygraph piston bore (8) or a semi-permeable membrane (3.2).

## Description

### FIELD OF THE INVENTION

The present invention relates to a device for culturing cells in adhesion; specifically, the device is optimized for the integration into a bench oxygraph reaction chamber and for the measurement of oxygen variations in the mono or co-culture cells layer.

### BACKGROUND ART

Mitochondria work in supplying cellular energy and are involved in many processes, such as several metabolic pathways, signaling, cellular differentiation, cell death, control of cell cycle and cell growth. Abnormal mitochondrial function occurs in a wide variety of diseases, such as diabetes and obesity, aging, cancer, cardiomyopathy, and neurodegeneration. Measurement of mitochondrial bioenergetics would provide valuable insight into several disorders and possibly lead to identification of targets for drug discovery. Generally these analysis are performed by means of oxygraph apparatus.

The conventional "reaction chamber oxygraph" instrumentation has a basic structure wherein a container (reaction chamber) made of plastic, stainless steel or glass has an hermetically closable upper end, and an open lower end interfaced with a cathode (made of platinum or gold) and an anode (made of silver or the like), covered by a film of aqueous potassium chloride solution, paper layer and a gas permeable membrane (made of polytetrafluoroethylene (PTFE), silicone polymer or the like), and sealed by means of a plastic O-ring. This model of oxygraph instrument is functional for measurement of the oxygen consumption of organelles/cells/tissues suspensions only ^{[1] [2] [3] [4] [5] [6]}.

In order to evaluate oxygen consumption of adherent cellular cultures, different methodology have been exploited. In this respect representative references to oxygen consumption measurement include several methodology approaches, specifically: optical probe, scanning electrochemical microscopy, electrochemical micro-sensor. Actually, in ^{[7] [8] [9] [10] [11] [12] [13]}, the authors described optical sensor platforms based on the changes in fluorescent dyes luminescence as a response to variations in oxygen concentration. Scanning electrochemical microscopy has been applied in a silicon chip with PDMS multichannels ^{[14]}. Electrochemical micro-sensors are usually based on Clark's micro-electrodes, where the oxygen undergoes cathodic reduction.

The devices of prior art can be classified into two group: external micromanipulator based and planar integrated micro-electrode. External micromanipulator based electrode has been used to make measurements of the pO2 gradient directly into the incubator. This open-air method is useful for studies on the metabolic properties of mono layers because the cells do not risk being damaged ^{[15]}, although this method may be less handy than systems described next. The second group of oxygen micro-sensors is represented by planar Clark micro-electrodes, basically a polydimethylsiloxane (PDMS) reservoir with an oxygen permeable membrane is interfaced with micro-electrodes at bottom. In particular Wu and collaborators used a PDMS conical container bound to a PDMS gas permeable membrane ^{[16] [17] [18]}. Similarly Park et al, employed this system, although their device was composed of three layers: a glass substrate with micro-electrodes, a chamber for the inner electrolyte divided into two compartments communicating by microchannels and a reservoir to store sample solution ^{[19]}.

In respect of reaction chamber, Ostrovidov et al. have made a PDMS microbioreactor to improve primary cells cultures, essentially structured as a thin PDMS porous membrane inserted between two microstructured PDMS layers provided of inlet and outlet holes, intended for a perfusion circuit device [20]. In respect of PDMS chemical surface treatment, for oxygen impermeabilization, the results obtained from Shiku and collaborators [21] have been considered as groundwork for development of the present oxygraph chamber insertable micro-culture device (MCD).

PDMS is the material of choice for biodevices micro fabrication mainly due to its UV-Vis transparency, well-characterized molding parameters, good solvent and chemical inertia and biocompatibility [30] ther materials with similar properties (except for high optical transmission in the visible wavelengths only) such as poly(methyl methacrylate) (PMMA) or polycarbonate (PC) can be used [31, 32].

WO200695480 discloses a cell culture chamber comprising a support, a semipermeable membrane, an upper compartment and a lower compartment both provided in the inside of the support and partitioned from each other by the semipermeable membrane, a culture medium feed flow path for feeding a culture medium into the lower compartment, a culture medium discharge flow path for discharging a culture medium in the lower compartment, and a perfusion flow path for perfusing a fluid through the upper compartment.

US20070212773 discloses an electrical signal measuring device that can measure electrical characteristics of cells in culture in real time, including a support body, a compartment in the support body, a semipermeable membrane, and electrodes, in which the compartment is divided by the semipermeable membrane into an upper compartment and a lower compartment; an upper electrode is provided in the upper compartment, and a lower electrode that faces the upper electrode and whose facing surface is in contact with the semipermeable membrane is provided in the lower compartment; and an upper perfusion channel and a lower perfusion channel are provided in the support body to separately perfuse fluid in the upper compartment and the lower compartment is disclosed. Said device is not used as an oxygraph chamber micro-adapter.

US2008009027 discloses a cell culture apparatus for culturing cells, which provides enhanced oxygen delivery and supply to cells without the need for stirring or sparging. Oxygen diffusion occurs on both sides of the culture vessel, top and bottom. A gaspermeable membrane that includes perfluorocarbons in its composition allows for the rapid, enhanced and uniform transfer of oxygen between the environment of cells or tissues contained in the cell culture container apparatus and the atmosphere of the incubator in which the cell culture apparatus is incubated. It discloses a single chamber apparatus and it is not used as an oxygraph chamber micro-adapter.

None of the cited documents discloses devices presenting an inlet at the top or a semi-permeable, namely permeable to O₂, CO₂ and N₂, membrane bonded at the upper end and/or an inlet at the bottom or a semi-permeable membrane bonded at the lower end.

A complex biochemical process is represented by fermentation, in particular wine fermentation is usually conducted by yeasts belonging to the species Saccharomyces cerevisiae. Industrial S. cerevisiae strains are highly specialized organisms, which have evolved to utilize to their full potential in the different environments.

During aerobic fed-batch growth for biomass production, yeast cells are exposed to oxidative stress (derived mainly through the aerobic metabolism of yeasts), hyperosmotic stress, ionic stress, etc. During the alcoholic fermentation and the industrial preparation, yeast cells are subjected to a number of adverse conditions, the most important being osmotic and ethanol stresses. The regulation of the stress response includes sensor systems and signal transduction pathways which result in the activation of the so-called stress response genes. A. Querol and colleagues [22] have reviewed, in this frame, about the adaptative genome rearrangements and interspecific hybridization between different species of Saccharomyces, screening that are conducted also in microvinification conditions.

Microvinification is a technique often used for experimental or scientific research but also for the screening of industrial strain and culture media. Just last year Schmidt, S.A. et al. [23] communicated the use of sub-millilitre culture volumes in microtiter plate (MTP) for self-induced anaerobic fermentation by means of Breathe Easy gas permeable membrane (Diversified Biotech, Boston, MA, USA) for sealing a commercial MTP, in particular they showed how to measure different parameter in fermentation performance, among this: measurement of MTP weight at the beginning and after the process in which yeast interact with sugars in the juice to create ethyl alcohol, and carbon dioxide (CO2 passes through the membrane, and so a loss in weight is registered).

On the other hand, different research groups exploited oxygraph technique (Oroboros Innsbruck, Austria, reaction chamber Oxygraph) for evaluation of oxygen consumption by Saccharomyces cerevisiae under enological conditions and non-respiratory oxygen consumption pathways in anaerobically-grown S. cerevisiae [24; 25] as well as the influence on oxygen consumption capacity during simulation of wine aging and the effect of different yeast strains and their culture conditions on the prevention of wine model solution browning by yeast lees [26, 27].

### SUMMARY OF THE INVENTION

The present invention relates to a semi-permeable micro-chamber system with the specific function of cell culture vessel, insertable in a reaction chamber oxygraph. It can be used for an accurate evaluation of dissolved oxygen concentration, at different time points, in order to quantify the oxygen consumption rate (OCR) of solutions in micro volumes, microbial cells in liquid or solid medium, and mainly for OCR of adherent cells cultures.

Actually, performing cellular OCR measurement by means of a reaction chamber oxygraph apparatus requires volumes of sample ≥ 200 µl and if working with anchorage dependent cells, to detach cells from the culturing substrate and re-suspend them into the apparatus' reaction chamber.

The oxygraph chamber insertable micro-culture device (MCD) of the invention allows performing OCR tests of volumes of investigated solution or cells suspensions and of cells cultured in adhesion in the same apparatus, avoiding cells trypsinization.

Moreover it permits evaluation of the respiratory efficiency on the same cells (i.e. following a pharmacological treatment), also after many days, with minimal risk of contamination or stress.

Furthermore, the culture membrane is removable and easy to lay down on a glass slide for auxiliary analysis.

To perform cellular OCR measurement, by means of a conventional oxygraph apparatus, it is necessary to detach adhering cultured cells from the adhesion substrate (plates, flask). As an alternative, the device of the invention, MCD, allows OCR tests on adhering cells, in the cited apparatus reaction chamber, without detaching them. Hence MCD makes possible more assays on the same cells (i.e. before and following a pharmacological treatment at different time-points) also after more days, with very low risk of contamination or stress.

Besides, the invention can be beneficial as new disposable platform in microvinification condition assessment, for the screening of industrial yeast strains and culture media. Specific arguments are represented by oxygen consumption evaluation in fermentation's aerobic phase (for cell growth) and in non-respiratory oxygen consumption pathways in anaerobically-grown Saccharomyces cerevisiae cells.

The present invention also relates to a method for measuring oxygen consumption rate in an oxygraph, comprising the steps of: culturing cells on a semi-permeable nanoporous membrane of a micro culture device (MCD) by the steps of inserting suspension cells in an upper or in a lower microchamber through suitable inlet and incubating the MCD at controlled atmosphere, i.e. 37°C, 5% CO₂, wherein cells sediment and adhere on the surface of the semi-permeable nanoporous membrane; measuring oxygen consumption rate by the steps of inserting the MCD in the oxygraph reaction chamber keeping it in contact with a Clark electrode disc and hermetically closing the reaction chamber; optionally adding a compound during said step of measuring by the steps of opening a micro cap of a oxygraph plunger and injecting a compound into MCD through the inlet and closing the micro cap; and running oxygen consumption rate measurement, being performable at different growing phases of cells.

The invention further refers to a method for analysing cells after measuring oxygen consumption rate, comprising the steps of: removing MCD from an oxygraph reaction chamber; performing cytochemical assay; disassembling MCD and recuperating the intermediate membrane; and performing morphological evaluation by microscope.

### DETAILED DESCRIPTION OF THE INVENTION

MCD permits oxygen consumption measurement on very small volumes and on adherent cells by means of reaction chamber oxygraph; so application of MCD to conventional instrumentation, permits to test both micro samples of solution/suspension and cells in adhering status. Additional advantages are here highlighted:
- small number of cells are required;
- low volume of sample and hence low amount of reagents are required;
- the system is disposable and avoids oxygraph chamber contamination;
- MCD allows running many measurements (at different time points) on the same cells; in fact, after the performed measurements it can be put back into the CO₂ incubator and applied to oxygraph's reaction chamber again;
- the ability of MCD to be disassembled, makes it versatile: it is feasible to evaluate oxygen consumption and to perform cytochemical assay and morphological evaluation on the same cells sample (allowing, i.e. for co-culture samples, comparative analysis or protein expression);
- MCD can be machined for reaction chamber oxygraphs of different firms, by changing shape and size of the steel molds;
- it may be utilized as adapter to oxygraphs of prior art (i.e. as those described in [16,17,18,19]).

The present invention therefore relates to a ready-to-use culture/co-culture microsystem, chemically modified for oxygen permeability and appositely machined to be housed within a conventional oxygraph reaction chamber. It is composed by a cylindrical chamber made of polydimethylsiloxane (PDMS) or polymethyl methacrylate (PMMA) or polycarbonate (PC) divided into two compartments, by a thin nanoporous membrane made of polydimethylsiloxane (PDMS) or polycarbonate (PC) or polytetrafluoroethylene (PTFE), where cells can be easily cultured. The cylindrical vessel has an inlet (or a nanoporous PDMS membrane for particular contamination sensitive cell culture) at the top and an inlet or a nanoporous PDMS membrane at the bottom to allow the passage of the gases during cell culture in CO2 incubator and the passage of oxygen to the Clark electrode disc, once MCD is inserted in the oxygraph reaction chamber to run a measurement.

The MCD is suitable for long time adhesion cultures and biochemical or biomolecular tests on cells. Moreover, once performing a single or several assays, on the same sample, it can be disassembled to separate and to use the membrane cell culture disc for further optical analysis of adhering cells on a microscope slide, also in fluorescence microscopy, due to PDMS optical properties (PDMS is optically transparent from 235 nm to the near-infrared).

The present invention will be now illustrated by means of the following non limiting figures, wherein:
- figure 1 shows four perspective views of two embodiments (left and right) of the MCD of the instant invention;

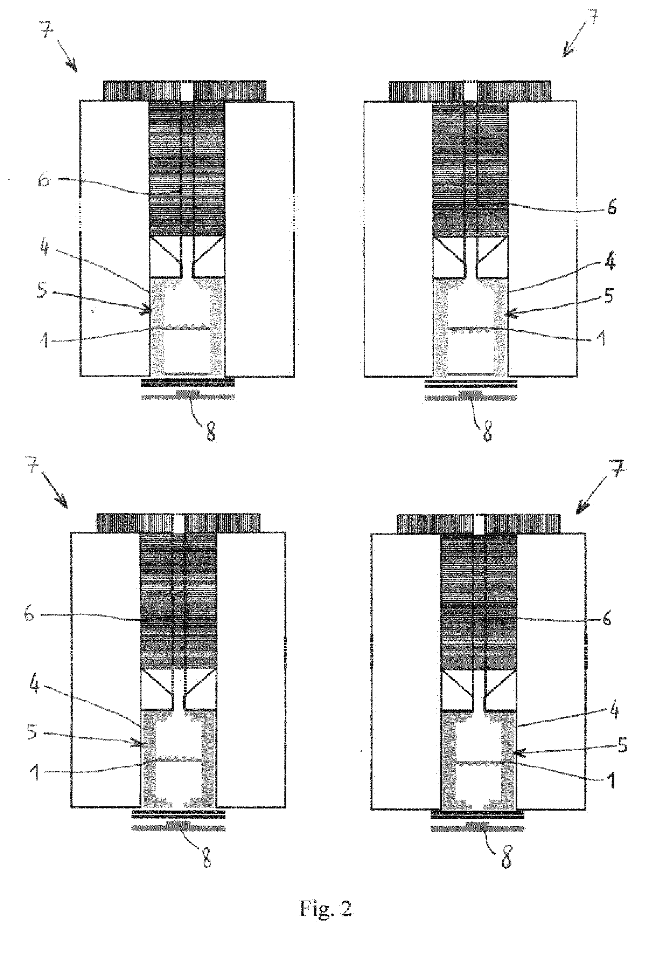

- figure 2 shows a longitudinal section of MCD alternative models applied to oxygraph reaction chamber;

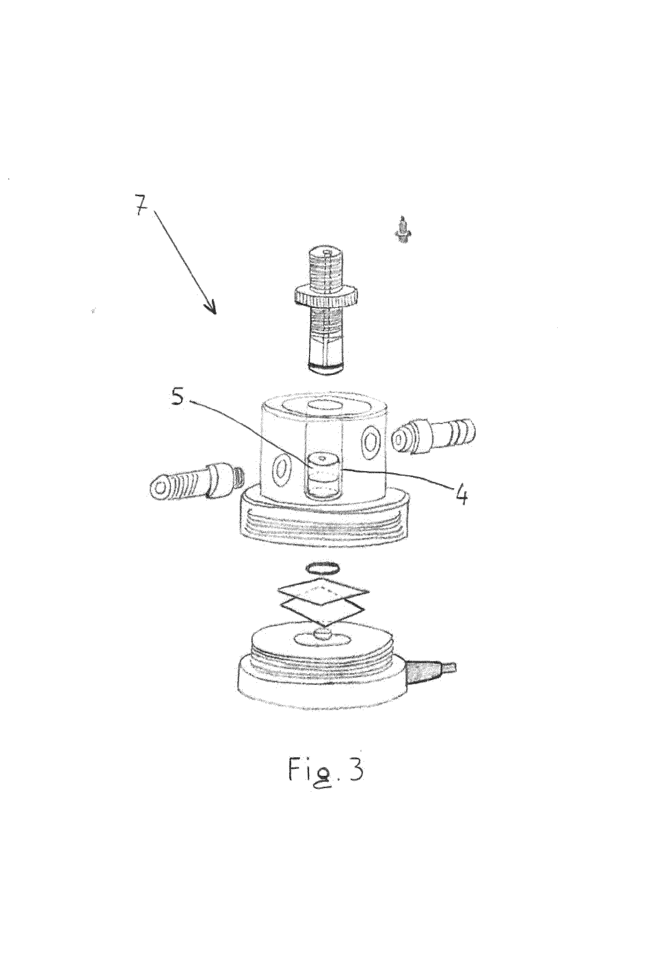

- figure 3 shows MCD within the oxygraph chamber;

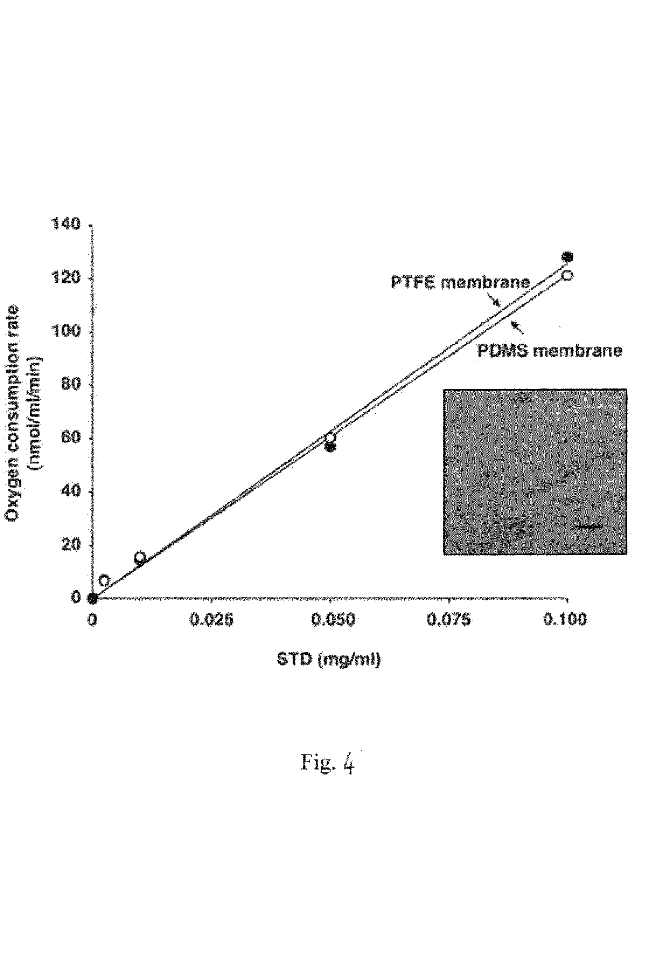

- figure 4 shows a graph relating to PDMS membrane oxygen permeability:
   PTFE or PDMS nanoporous membrane was applied to the electrode dome and a saturated KC1 solution was maintained between the electrode and the membrane. After calibration of the system, oxygen content was measured in the oxygraph chamber containing 1 ml of distilled water or 1 ml of a sodium dithionite (SDT) solution. Oxygen consumption was calculated as the rate of decrease in oxygen concentration and was expressed as nmol O₂ x ml⁻¹ x min⁻¹. The inset shows a SEM image of PDMS porous membrane. PDMS prepolymer was composed of PDMS base and curing agent at a ratio of 10/1 (w/w); water was used as porogen at 50% (w/v). The spin coating condition for fabricating the membrane was set to 1500 rpm for 30 s, to obtain a thickness under 100 µm, as assessed by surface profilometer scansion. Pores dimensions were estimated by SEM imaging and a range from 50 to 250 nm was found for major diameter (Scale bar 200 nm);

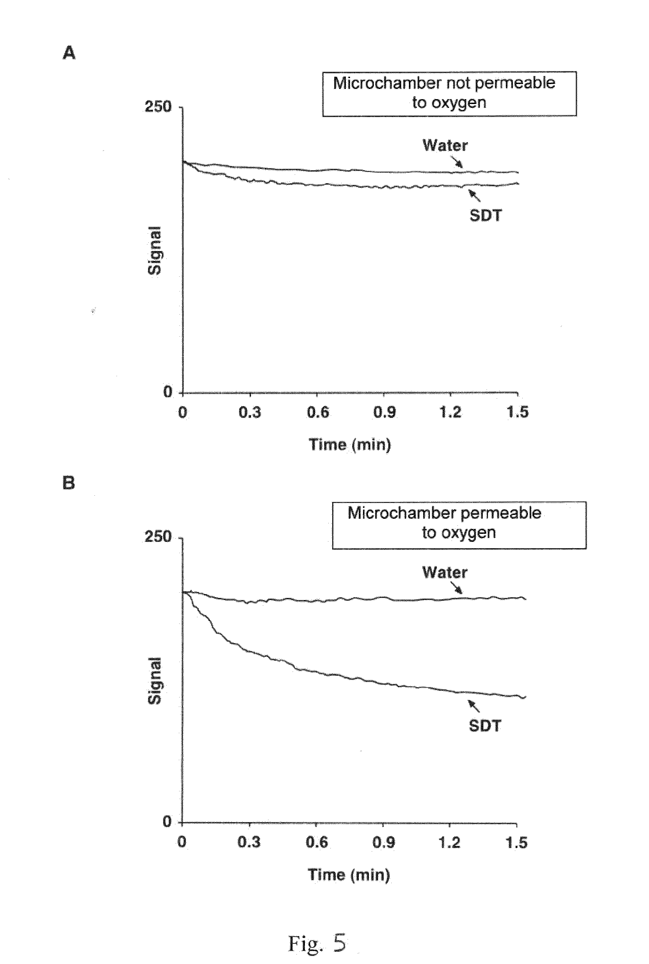

- figure 5 shows graphs relating to oxygen diffusion in microchamber (Plasma/BSA treatment for PDMS surface O₂ impermeabilization); microchamber was placed into the oxygraph reaction chamber and was filled with 100 ml of distilled water or with 100 ml of SDT 0.1 mg/ml. Oxygen concentration in the oxygraph chamber was monitored over time. Case A refers to closed microchamber treated for oxygen impermeabilization, whereas case B relates to Microchamber treated for oxygen impermeabilization (oxygen may diffuse through the inlet or through the semipermeable membrane at the bottom);

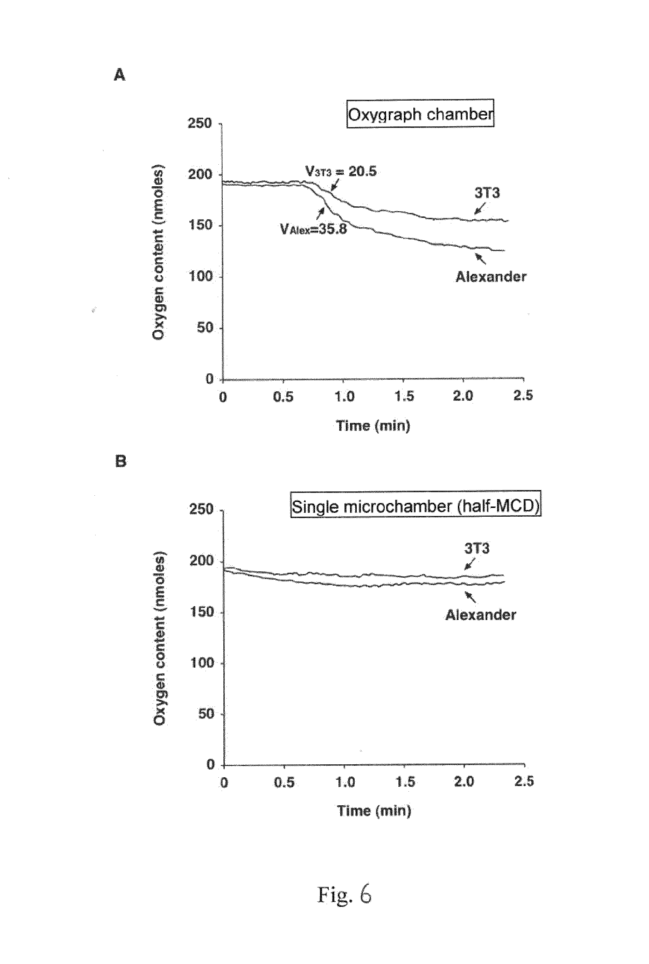

- figure 6 shows graphs relating to determination of oxygen consumption from cells suspended in oxygraph chamber or from adherent cells on O₂ permeable membrane at the bottom of a single microchamber (placed within oxygraph). In case A, 9x10³ cells (3T3 or Alexander) were stirred in the oxygraph reaction chamber at 37°C in L15 medium. The rate of oxygen uptake (V) is expressed as nmol O₂ x ml⁻¹ x min⁻¹. In case B, 9x10³ cells (3T3 or Alexander) were seeded on the flat O₂ permeable membrane of a single microchamber (the upper chamber of the cylinder). After 24 h of incubation, microchamber was placed into the oxygraph reaction chamber and oxygen concentration was monitored over time; it is evident that adhering cells, in narrow contact with the electrode disc, interfere with measurement, so a reservoir-type chamber is necessary for the system;

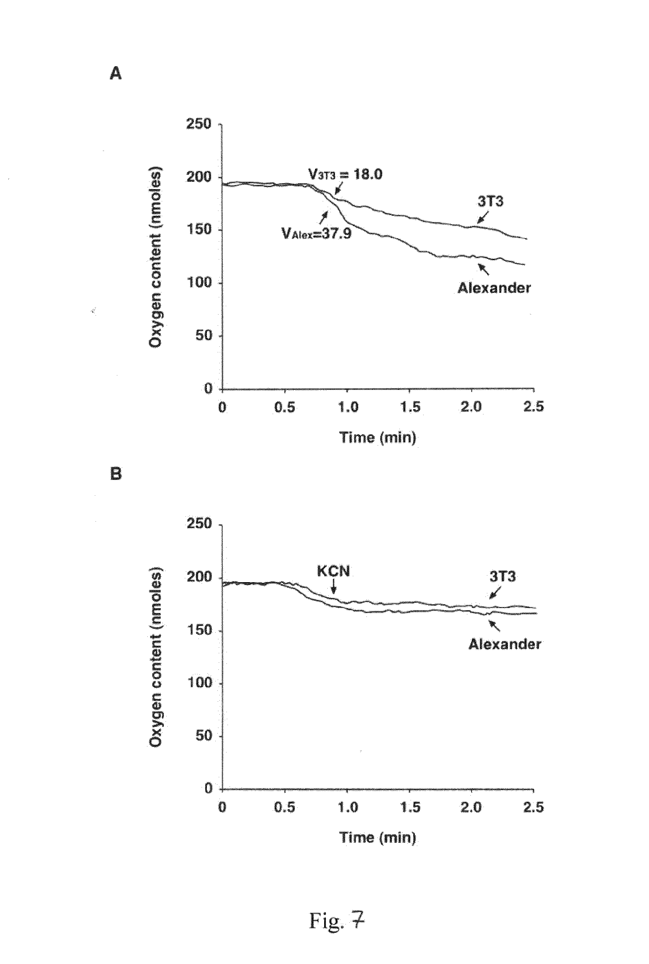

- figure 7 shows graphs relating to determination of respiration from cells seeded on the middle membrane of a double microchamber (MCD) and KCN inhibition test: 9x10³ cells (3T3 or Alexander) were seeded on the flat O₂ permeable membrane in the middle of MCD. After 24 h of standard incubation, DMEM was replaced with L15, and MCD was placed into the oxygraph reaction chamber where oxygen concentration was monitored (case A); cell respiration was inhibited by the addition of KCN 5 mM (case B). Oxygen consumption rate (V) is expressed as nmol O₂ x min⁻¹;

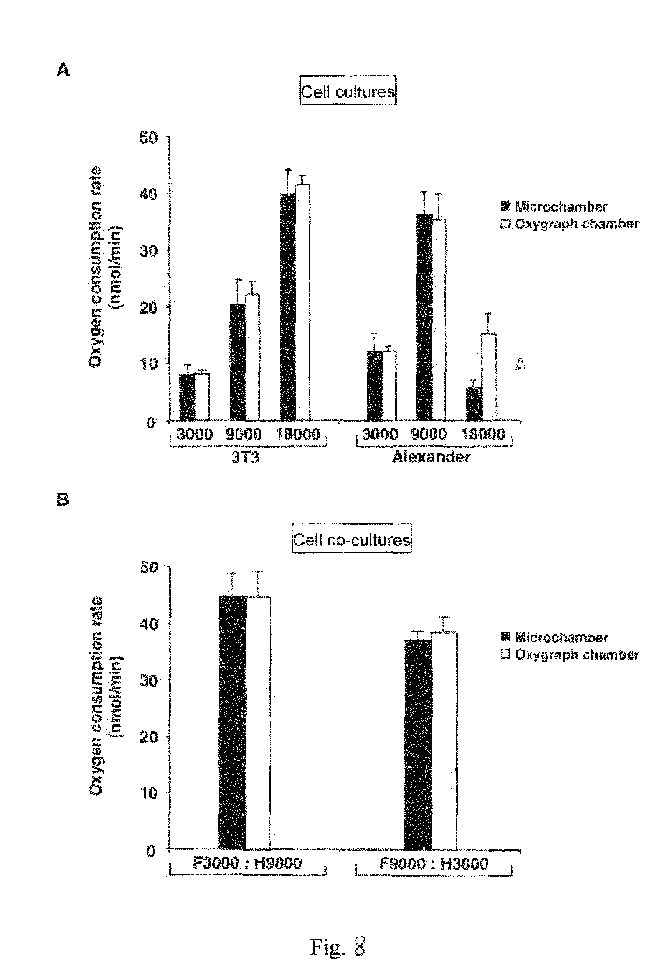

- figure 8 shows graphs concerning determination of respiration from cells seeded as mono and co-cultures: MCD with cell monocultures (case A) or monolayer co-coltures (case B) were placed into the oxygraph chamber and oxygen concentration was monitored. Oxygen consumption was calculated as the rate of decrease in oxygen concentration. Data are the mean ± SD (n=3). In Alexander liver cancer cell line, the value of O₂ consumption, at high cell density (64 x 10³ cells/cm²), is lower, both in MCD and in the standard oxygraph chamber, similar data are reported in literature by independent studies performed on primary hepatocytes. Low correlation between MCD and standard reaction chamber in case A (Δ) is probably due to some clogged spots on low porous membrane by cells clusters, typical adhesion pattern of this cell line;

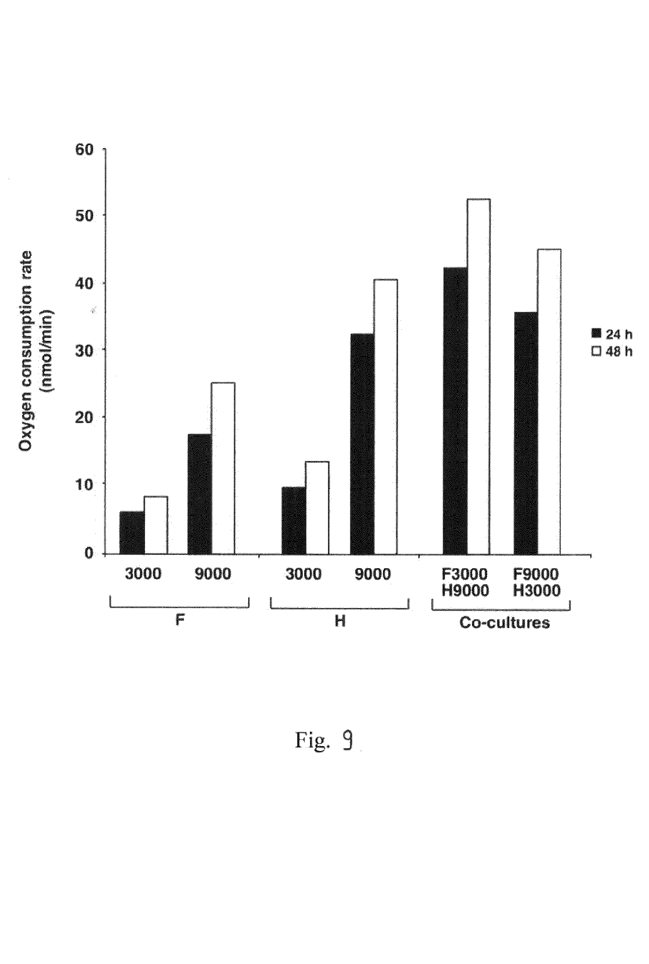

- figure 9 shows a graph relating to determination of respiration from cell cultures and co-cultures after 24 h - 48 h of incubation: MCD with cell monocultures or co-cultures were placed into the oxygraph chamber and oxygen consumption was calculated. After 24 h of standard incubation, medium was replaced with L15 and a first measurement was done; promptly, tested microchambers were removed from oxygraph reaction chamber, and left, in DMEM, in a CO₂ incubator at 37°C, for other 24 h for successive measurement;

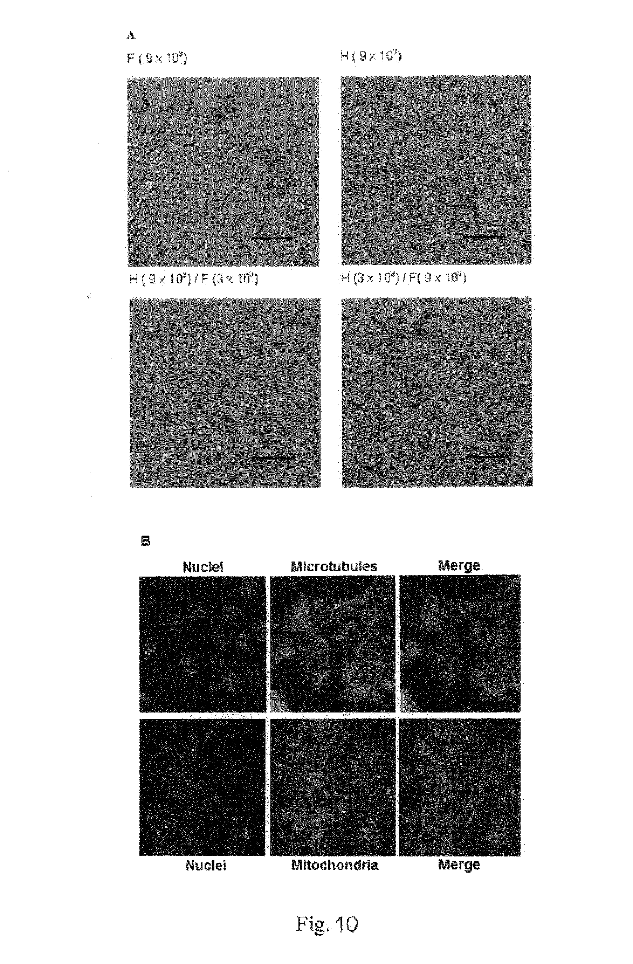

- figure 10 shows, in case A, images regarding adhering cells morphological evaluation by optical microscopy: 3 x10³ and 9 x10³ cells (3T3 or Alexander) were seeded on the intermediate O₂ permeable membrane, in mono and in co-culture with different ratio. After 48 h of incubation at 37°C, 5% CO₂, MCD was disassembled and membrane disc placed on microscope slide for optical evaluation (scale bar 100 µm). Cytochemical test may be performed on these cells monolayer (opportunely within the microchamber); what is more, PDMS elasticity preserves, in a more efficient manner, cells adhesion, throughout staining steps. In case B, images are shown relating to optical evaluation by fluorescence microscopy: 9 x10³ cells (3T3) were seeded on the intermediate membrane in MCD and after 48 h of incubation at 37°C, 5% CO₂, cells were double stained with Mitotracker Green and DAPI (specific for mitochondria and nuclei, respectively) and anti-β Tubulin antibody conjugated with FITC fluorophore and DAPI (specific for cytoskeleton Tubulin and nuclei, respectively) and visualized at the fluorescence microscope (obj. 20x and 40x);

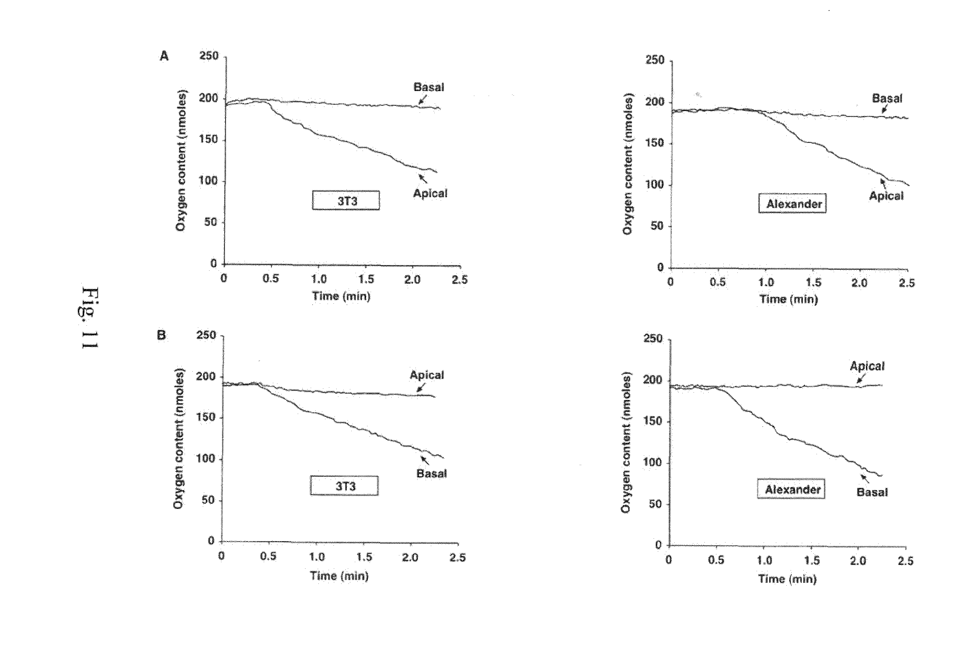

- figure 11 relates to oxygen diffusion evaluation: oxygen uptake from cells cultured on the apical or luminal side of the intermediate semi permeable, as in case A, or oxygen impermeable membrane, as in case B, in a double microchamber (MCD). In this semi-permeable system, electrode disc is able to measure with efficiency O₂ dissolved in the closer chamber. As for case A relating to oxygen permeable membrane, if cells adhere on the apical side, oxygen is consumed sooner in the upper chamber, and, promptly, the consumed molecules are replaced by those dissolved in the lower chamber, strictly in contact with the electrode disc; this is due to the gas diffusion from a more concentrated compartment to that with a lower concentration; hence the cathode reduces a number of oxygen nanomoles that is proportional to those consumed in the upper chamber (by cells). On the other hand, if cells adhere on the basal side, oxygen is consumed sooner in the lower chamber and promptly, the consumed molecules are replaced by those dissolved in the upper chamber where now O₂ is more concentrated; hence the cathode reduces the same nanomoles of oxygen like those present at time zero. The evidence of this dynamic is provided by an additional matching test, performed with an intermediate oxygen impermeable membrane, and it is represented in B. As for case B relating to oxygen impermeable membrane, if cells are on the apical side, oxygen is consumed in the upper chamber and the consumed molecules are not replaced by those dissolved in the lower one because of membrane impermeability; hence the cathode reduces the oxygen nanomoles present at time zero in the lower chamber, and no consumption is registered. Then, if cells adhere on the basal side and oxygen is consumed, as the intermediate membrane is impermeable, no molecules are replaced in the lower chamber, from the upper compartment, and the oxygen consumption is registered;

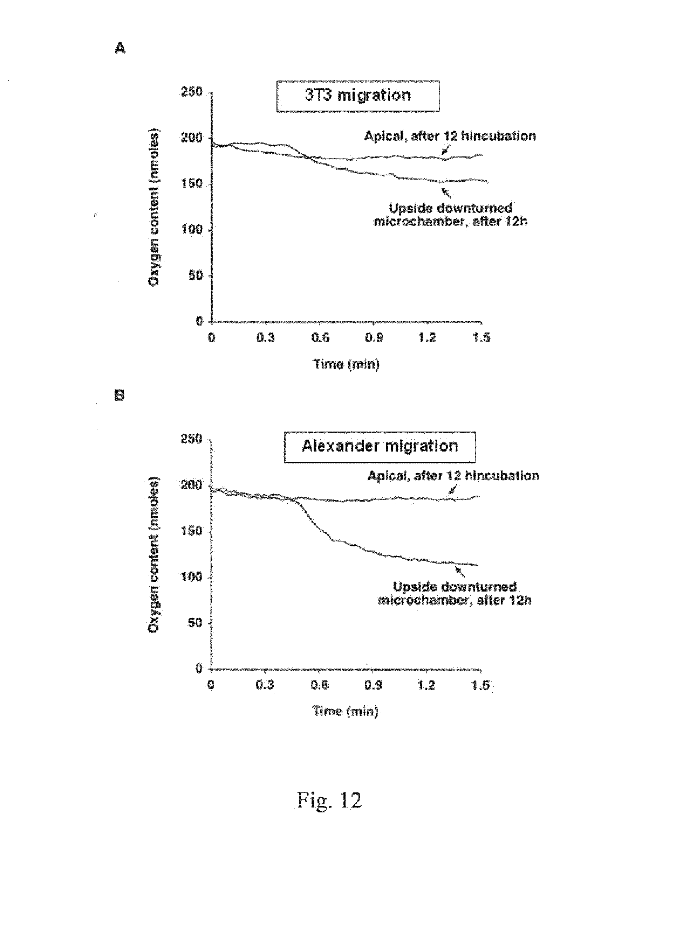

- figure 12 relates to migration assay, determination of respiration from both 3T3, as in case A, and Alexander cells, as in case B. 9x10³ cells (3T3 or Alexander) were seeded on the apical side of the intermediate O₂ permeable membrane within MCD; after few hours, cell migration was induced by changing serum concentration (lower (2%) and higher (10%), respectively in the upper and lower chamber) and, behind 12 h of incubation, MCD was placed into the oxygraph chamber and oxygen concentration was monitored. Measurements were done standing MCD up straight (cells were transmigrated in the luminal, or basal side and hence none O₂ concentration decrement was assessed) and turning it upside down (transmigrated cells are now exposed in the upper chamber and O₂ concentration decrement was assessed. Designed mixture PDMS / curing agent / water carries on high elasticity and plasticity, so that cells are able to trans migrate even if porous are smaller than those of the commercial *transwell* membrane. Cells trans migration was assessed by detachment and cell counting on hemocytometer. The oxygraph chamber insertable micro-culture device 5 can be produced using, for example, moulding standard techniques, spin coating, conformal contact for the adhesion of the PDMS microchambers with the PDMS membrane.

The model described, potentially, will allow to develop MCD application in tissue regeneration studies, for bio-scaffold efficiency evaluation, also in terms of cell migration from a side to the opposite one, in function of scaffold surface chemical functionalization, expressly studied to promote or inhibit cell migration in function of a specific tissue to regenerate.

### EXAMPLE

Referring to figures, the micro-culture device (MCD) 5 is totally fabricated in polydimethylsiloxane (PDMS). A PDMS/curing agent mixture (1:10 w/w) is casted in appositely fabricated steel molds and cured at 70°C for 90 min. Essentially, the micro-culture device (MCD) 5 is composed by three elements: oxygen-permeable nanoporous membrane 1 for cell culture, an upper microchamber 2 and a lower reservoir-type microchamber 3. The system will be described herein in details.

A PDMS/curing agent mixture (10:1 w/w) and water (as a porogen agent at a ratio of 50% water and 50% polymer base), is stirred for 30 min, spread on glass substrate by spin coating (1500 rpm, for 20 s) and then cured at 70°C for 30 min. The obtained porous membrane, detached from glass slide, results of 80 µm thickness, with porous ranging from 50 to 250 nm in diameter. In order to be suitable for cell culturing, the semipermeable nanoporous membrane 1 is then subjected at a plasma oxygen treatment by a plasma hasher (5 min, 20% O₂, 100W, 40 KHz) and incubated with 0,01% w/v poly-L-Lysine or extra cellular matrix protein for 30 min; this treatment facilitates the next cells adhesion on the polymeric membrane.

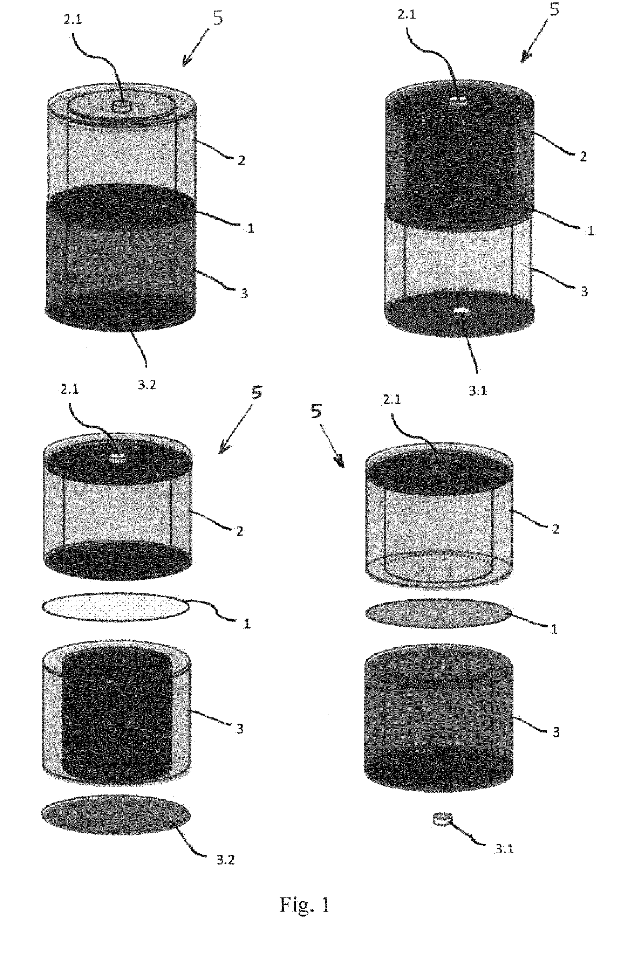

Polymerized microchambers are realized as hollow cylinders of 8 mm height (or less, or more, if required), 9 mm outer diameter, 6 mm inner diameter (cylinder side walls of 1,5 mm wide); the upper microchamber 2 presents, on the top, a central inlet 2.1 coaxially aligned with oxygraph plunger precision bore 6 of 1 mm inner diameter, and it is intended for solutions injection by means of a syringe, at some stage during evaluation; alternatively, it has a semi-permeable membrane bonded at the upper end. The lower microchamber 3, in the same way, has at the lower end a central inlet 3.1 positioned at the bottom and coaxially aligned with oxygraph piston bore, like the upper one (see figure 1), or a semi-permeable membrane 3.2 (see figure 1). The inlets conformed as telescopic sub micro-cylinders are obtained by a capillary placed on the cast and upholded, over the time of PDMS mixture pouring and polymerization, by means of an expressly fabricated mini column holder. The upper microchamber 2 and the lower microchamber 3 are subjected to a surface chemical process (plasma hasher (5 min, 20% O₂, 100W, 40 KHz) and 1h incubation with 0,01% w/v bovine serum albumin (BSA)) in order to avoid random gas exchange, and purposely address oxygen toward electrode disc.

After the above mentioned surface treatments the three elements are assembled by conformal contact with a PDMS thin film between the microchambers and the membrane, and cured at 70°C for 15-30 min. The whole system is autoclavable or easily sterilized by ethanol 70% and sterile double distilled water washing steps. Preferably, the invented system, during cells incubation, is placed and closed in a standard sterile well plate, to minimize microorganisms contamination; for this purpose, in order to well close the tray and the lid of the commercial multiwell plate, lower MCD can be realized.

As proof of principle, the MCD 5 has been applied to oxygraph chamber 4 of an oxygraph 7, to measure the OCR of two independent cells lines, seeded and allowed to growth in mono or co-culture, on the intermediate membrane in MCD, and the given results (see figures) are consistent to those obtained by standard tests performed on cells in agitation in the oxygraph reaction chamber barely. Indeed, MCD lets to perform OCR tests of adhering cells without detaching them, allowing different subsequent evaluations of the same sample, i.e. following a pharmacological test, after one or more days of incubation, with modest risk of contamination or stress.

Furthermore, after OCR evaluation, thanks to PDMS inertia to many chemicals used in cytochemistry, and thanks to elastomer plasticity and optical transparency (λ ≥ 235 nm), biochemical assay (i.e. MTT test) or cyto- and immunochemical evaluation can be performed within the invented device. Then MCD can be easily disassembled in the constituent modules 1, 2, 3 and, after this, the microchambers 2, 3 can be discarded and the intermediate membrane with the assayed cells still adhering on, can be settled on a microscope slide for optical imaging in transmission and fluorescence, as showed in figures.

Functional forms, basically, include MCD composed as:
- inlets 2.1, 3.1 on top and bottom of the MCD 5, fabricated as an inner subcylinder telescopic bore and designed to not pour out any liquids. Under operating in static conditions the inlet is small enough (1 mm in diameter) that it is impossible for the fluid to flow through the hole at the work temperature (20-37 °C); PDMS hydrophilicity, after chemical surface treatment, results in stronger adhesiveness between water molecules and PDMS walls. Moreover the laws and principles of hydrostatic suggest that in a small capillary hole, liquid surface tension pressure is greater than pressure of the liquid, preventing any fluid leakage. This model is also practical for sample handling;
- inlet 2.1 on the top and semi-permeable membrane 3.2 at the bottom. The semi-permeable membrane 3.2 at the bottom does not alter the efficiency of oxygen diffusion towards the electrode and, additionally, prevents contamination acting as a shielding nanoporous filter between sensing system and the lower microchamber; it is, mainly, intended for multiple test on the same sample, and for cell lines very sensitive to contamination, such as primary culture of somatic or stem cells.

In both these designs, a PDMS made bracket horse is useful, but not mandatory, as open pedestal insert for gas free exchange during cell incubation in CO₂ incubator; finally, within these configurations, the bracket horse insert should optimise gas diffusion through cell culture layer (as aforementioned in WO200806104, [29]). As usually, entry of CO₂, from the incubator atmosphere into a cell culture plate, involves a loosely fitting lid over the plate in excluding particulate contaminants from entering the plate chamber(s), but allows gas exchange between the incubator atmosphere and the atmosphere within the plates. Correspondingly, for cell culture flasks, a cap is provided with a gas permeable membrane or filter thereby allowing for gas exchange, and not particulate contaminants entry.

Possible alternative forms include an optimised intermediate porous membrane and inception of flat microporous bio-scaffold as intermediate insert, in the place of the PDMS porous membrane.

In a first possible alternative form, an optimised porous membrane fabricated by photolithographic techniques, with the aim to control pores geometry and distribution. Photolithography is a method used in microfabrication to transfer a pattern onto a surface, exploiting chemical and physical processes. This is possible by depositing a photoresist (photosensitive polymer) on the substrate and exposing it to UV light, inducing chemical changes that allows to remove the photoresist by a developer solvent. Subsequently, the layer of the substrate in the areas that are not protected by photoresist is removed by etching process, utilizing chemical agents attaining the final mold. Specifically, to realize a porous membrane, a mold with embossed micropillars is required, to apply and cure the polymer on. The polymer membrane removed from the mold results in a regular geometric conformation and distribution of the desired pores.

This option is related also to the application of MCD for high density trans-membrane co-culture (i.e. *in vitro* blood brain barrier co-culture), or for cells separation/migration devices, in which a regular porosity is necessary to allow a free gas exchange also in the presence of high cell density onto the membrane surface, in case of application for high density trans-membrane co-culture; to warrant a precise porosity, specific for different cell lines (with different cells diameter), in case of application for cells separation/migration devices.

A second possible alternative form provides for inception of flat microporous bio-scaffold as intermediate insert, in the place of the PDMS porous membrane, intended as a novel application in tissue engineering scaffold characterization. Specifically intended for oxygen diffusion dynamic evaluation into the cultured matrix, and, hence for assessing matrix efficiency as bio-scaffold for tissue regeneration, besides, this application refers to cells migration test of chemically functionalised bio-scaffold.

### APPLICATIONS

The present invention is intended for *in vitro* applications, mainly in the field of biology (physiological and disease models), regenerative medicine (stem cells and tissue engineering), bio- and nanobiotechnology, pharmacology (drug discovery) and tossicology.

Essentially MCD makes possible to analyse micro volumes of samples and cells in adhesion, into the oxygraph reaction chamber. This ability can be exploited for biochemical assays on cells in mono- and/or co-culture (also at different time points) and subsequent imaging of the same sample.

In particular it can be applied for:
- estimation of the response to toxicants and the correlation of response to exposure with the etiology and progression of dysfunction (by means of respiration and immunocytochemical assays);
- development and characterization of chemical, structural, and biological properties of nanostructured assemblies/materials to advance functional tissue engineering;
- drug delivery development and nano-formulations of existing therapeutic drugs acting at subcellular level (e.g., mitochondrial)-targeting;
- development and/or monitoring of mitochondrial gene delivery, a novel strategy for curing a mitochondrial disease.

Moreover it can be applied for:
- estimation of oxygen consumption by Saccharomyces cerevisiae under enological conditions in order to evaluate the effect on fermentation kinetics;
- colony morphology evaluation in efficient yeast strain screening;
- yeast fermentation by weight loss measurement (CO₂ escape).

### REFERENCES

1. Wieser, W. and G. Krumschnabel, Hierarchies of ATP-consuming processes: direct compared with indirect measurements, and comparative aspects. Biochem J, 2001. 355(Pt 2): p. 389-95.
2. Marchandeau, J.P. and G. Labbe, Evaluation of mitochondrial respiration in cultured rat hepatocytes. Methods Mol Biol, 2011. 691: p. 243-53.
3. Hughey, C.C., et al., Respirometric Oxidative Phosphorylation Assessment in Saponinpermeabilized Cardiac Fibers. J Vis Exp, 2011(48).
4. Ferramosca, A., et al., Oxygen uptake by mitochondria in demembranated human spermatozoa: a reliable tool for the evaluation of sperm respiratory efficiency. Int J Androl, 2008. 31(3): p. 337-45.
5. Villani, G. and G. Attardi, Polarographic assays of respiratory chain complex activity. Methods Cell Biol, 2007. 80: p. 121-33.
6. Pipinos, II, et al., Abnormal mitochondrial respiration in skeletal muscle in patients with peripheral arterial disease. J Vasc Surg, 2003. 38(4): p. 827-32.
7. Sud, D., et al., Optical imaging in microfluidic bioreactors enables oxygen monitoring for continuous cell culture. J Biomed Opt, 2006. 11(5): p. 050504.
8. Deshpande, R.R., et al., Microplates with integrated oxygen sensors for kinetic cell respiration measurement and cytotoxicity testing in primary and secondary cell lines. Assay Drug Dev Technol, 2005. 3(3): p. 299-307.
9. Beckers, S., et al., High throughput, non-invasive and dynamic toxicity screening on adherent cells using respiratory measurements. Toxicol In Vitro, 2010. 24(2): p. 686-94.
10. Gao, F.G., A.S. Jeevarajan, and M.M. Anderson, Long-term continuous monitoring of dissolved oxygen in cell culture medium for perfused bioreactors using optical oxygen sensors. Biotechnology and Bioengineering, 2004. 86(4): p. 425-433.
11. Cho, C.H., et al., Oxygen uptake rates and liver-specific functions of hepatocyte and 3T3 fibroblast co-cultures. Biotechnol Bioeng, 2007. 97(1): p. 188-99.
12. Oppegard, S.C. and D.T. Eddington, Device for the control of oxygen concentration in multiwell cell culture plates. Conf Proc IEEE Eng Med Biol Soc, 2009. 2009: p. 2097-100.
13. Papas, K.K., et al., A stirred microchamber for oxygen consumption rate measurements with pancreatic islets. Biotechnology and Bioengineering, 2007. 98(5): p. 1071-1082.
14. Torisawa, Y.S., et al., Multi-channel 3-D cell culture device integrated on a silicon chip for anticancer drug sensitivity test. Biomaterials, 2005. 26(14): p. 2165-72.
15. Mamchaoui, K. and G. Saumon, A method for measuring the oxygen consumption of intact cell monolayers. American Journal of Physiology-Lung Cellular and Molecular Physiology, 2000. 278(4): p. L858-L863.
16. Wu, C.C., et al., Fabrication of miniature Clark oxygen sensor integrated with microstructure. Sensors and Actuators B-Chemical, 2005. 110(2): p. 342-349.
17. Wu, J., et al., The effect of the closely-spaced working and auxiliary electrodes on the performance of electrochemical oxygen sensor. Electroanalysis, 2007. 19(18): p. 1939-1943.
18. Wu, C.C., et al., A Clark-type oxygen chip for in situ estimation of the respiratory activity of adhering cells. Talanta. 81(1-2): p. 228-34.
19. Park, J., Y.K. Pak, and J.J. Pak, A microfabricated reservoir-type oxygen sensor for measuring the real-time cellular oxygen consumption rate at various conditions. Sensors and Actuators B-Chemical, 2010. 147(1): p. 263-269.
20. Ostrovidov, S., et al., Membrane-based PDMS microbioreactor for perfused 3D primary rat hepatocyte cultures. Biomedical Microdevices, 2004. 6(4): p. 279-287.
21. del Alamo, M. and I. Nevares, Measurement of dissolved oxygen during red wines tank aging with chips and micro-oxygenation. Analytica Chimica Acta, 2008. 621(1): p. 68-78.
22. Querol, A., et al., Adaptive evolution of wine yeast. International Journal of Food Microbiology, 2003. 86(1-2): p. 3-10.
23. Schmidt, S.A., et al., Microvinification-how small can we go? Applied Microbiology and Biotechnology, 2011. 89(5): p. 1621-1628.
24. Rosenfeld, E., et al., Oxygen consumption by anaerobic Saccharomyces cerevisiae under enological conditions: Effect on fermentation kinetics. Applied and Environmental Microbiology, 2003. 69(1): p. 113-121.
25. Rosenfeld, E., et al., Non-respiratory oxygen consumption pathways in anaerobically-grown Saccharomyces cerevisiae: evidence and partial characterization. Yeast, 2002. 19(15): p. 1299-321.
26. Salmon, J.M., C. Fornairon-Bonnefond, and J.P. Mazauric, Interactions between wine lees and polyphenols: Influence on oxygen consumption capacity during simulation of wine aging. Journal of Food Science, 2002. 67(5): p. 1604-1609.
27. Salmon, J.M., et al., Effect of Different Yeast Strains and Their Culture Conditions on the Prevention of Wine Model Solution Browning by Yeast Lees. Journal of Agricultural and Food Chemistry, 2009. 57(9): p. 3771-3779.
28. Stambuk, B.U., et al., Switching the mode of sucrose utilization by Saccharomyces cerevisiae. Microbial Cell Factories, 2008. 7.
29. WO200806104: ENHANCED OXYGEN CELL CULTURE PLATFORMS UNIV MIAMI;
30. George M. Whitesides, Emanuele Ostuni Shuichi Takayama Xingyu Jiang and Donald E. Soft lithography in biology and biochemistry. Ingber Annu. Rev. Biomed. Eng. 2001. 3:335-73
31. Chia-Wen Tsao Æ Don L. DeVoe, Bonding of thermoplastic polymer microfluidics. Microfluid Nanofluid (2009) 6:1-16
32. Xinyu Huang and William Synthesis and Characterization of PMMA Nanocomposites by Suspension and Emulsion Polymerization. J. Brittain Macromolecules 2001, 34, 3255-3260.

## Claims

1. A micro culture device (5) to be inserted in a chamber (4) of an oxygraph (7), the micro culture device being divided into an upper micro-chamber (2) and a lower microchamber (3) by a semi-permeable nanoporous membrane (1), suitable for acting as adhering support for cells,
**characterized in that**:
a) the upper microchamber (2) presents at its upper side either an inlet (2.1) coaxially aligned with an oxygraph plunger precision bore (6), or a semi-permeable membrane; and
b) the lower microchamber (3) presents at its bottom side either an inlet (3.1) coaxially aligned with an oxygraph piston bore (8) or a semi-permeable membrane (3.2).

2. The micro culture device according to claim 1, **characterized in that** it is essentially of cylindrical shape.

3. The micro culture device according to claim 1 or 2, **characterized in that** the semipermeable nanoporous membrane (1) is removable.

4. The micro culture device according to any one of claims 1 to 3, further comprising a PDMS made bracket horse as open pedestal insert for gas free exchange during cell incubation in CO₂ incubator.

5. The micro culture device according to any one of previous claims being made of polydimethylsiloxane (PDMS), or polycarbonate (PC), or polymethylmethacrylate (PMMA).

6. The micro culture device according to any one of previous claims wherein the semi-permeable nanoporous membrane (1) is preferably made of polydimethylsiloxane (PDMS), or polytetrafluoroethylene (PTFE), or the like.

7. An oxygraph comprising the micro culture device according to any one of previous claims.

8. Use of the oxygraph according to claim 7 for oxygen consumption rate measurement of cells adhered to the semi-permeable nanoporous membrane (1) of the micro culture device.

9. A method for measuring oxygen consumption rate in an oxygraph, **characterized in that** it comprises the steps of:
● culturing cells on the semi-permeable nanoporous membrane (1) of the micro culture device according to any one of claims 1 to 6 by the steps of:
- inserting suspension cells in the upper or in the lower microchamber through suitable inlet;
- incubating the MCD at controlled atmosphere, i.e. 37°C, 5% CO₂, wherein the cells sediment and adhere on the surface of the semipermeable nanoporous membrane (1);
● measuring oxygen consumption rate by the steps of:
- inserting the MCD in the oxygraph reaction chamber keeping it in contact with the Clark electrode disc;
- hermetically closing the reaction chamber;
● optionally adding a compound during said step of measuring by the steps of:
- opening a micro cap of the oxygraph plunger and injecting the compound into MCD through the inlet;
- closing the micro cap;
● running oxygen consumption rate measurement.

10. Method according to claim 9, **characterized in that** the step of measuring oxygen consumption rate is performed at different growing phases of cells.

11. Method for analysing cells after measuring oxygen consumption rate, **characterized in that** it comprises the steps of:
- removing MCD from the oxygraph reaction chamber;
- performing cytochemical assay;
- disassembling MCD and recuperating the intermediate membrane;
- performing morphological evaluation by microscope.
